# EUROPEAN PATENT SPECIFICATION

(11) **EP 3 212 240 B1**
(45) Date of publication and mention of the grant of the patent: **31.07.2024**
(21) Application number: 14904920.7
(22) Date of filing: 30.10.2014
(51) Int. Cl.: A61K 49/22, A61K 49/04, A61K 49/08, A61K 49/12

(54) **LIPID ENCAPSULATED GAS MICROSPHERE COMPOSITIONS AND RELATED METHODS**
LIPIDVERKAPSELTE GASMIKROSPHÄRENZUSAMMENSETZUNGEN UND ENTSPRECHENDE VERFAHREN
COMPOSITIONS DE MICROSPHÈRES GAZEUSES ENCAPSULÉES DANS DES LIPIDES ET PROCÉDÉS ASSOCIÉS

(43) Date of publication of application: 06.09.2017
(73) Proprietor: Lantheus Medical Imaging, Inc., North Billerica, MA 01862 (US)
(72) Inventor: ROBINSON, Simon P., Stow, MA 01775 (US); NGUYEN, Nhung Tuyet, Westford, MA 01886 (US); SIEGLER Robert, W., Chelmsford, MA 01824 (US)
(74) Representative: Carpmaels & Ransford LLP
(86) International application number: PCT/US2014/063267
(87) International publication number: WO 2016/068961

(56) References cited:
- WO-A1-98/51284
- WO-A2-01/08711
- WO-A2-2013/013067
- WO-A2-2013/013067
- US-A- 5 656 211
- US-A- 5 922 304
- US-A1- 2013 309 174
- US-A1- 2013 309 174
- SARKAR, K. ET AL.: "Growth and dissolution of an encapsulated contrast microbubble: effects of encapsulation permeability", ULTRASOUND IN MEDICINE & BIOLOGY, vol. 35, no. 8, 2009, pages 1385 - 1396, XP026321838, DOI: doi:10.1016/j.ultrasmedbio.2009.04.010
- HETTIARACHCHI, K. ET AL.: "On-chip generation of microbubbles as a practical technology for manufacturing contrast agents for ultrasonic imaging", LAB ON A CHIP, vol. 7, no. 4, 2007, pages 463 - 468, XP002490904

## Description

### SUMMARY OF THE INVENTION

The invention provides compositions comprising lipid-encapsulated gas microspheres and methods of their production and of their use. The compositions of the invention are compositions used to form an ultrasound contrast agent or are ultrasound contrast agent compositions. The compositions of the invention comprise a lipid solution comprising DPPA, DPPC and PEG5000-DPPE, and a perfluorocarbon gas, in a container, wherein the lipid solution comprises 0.1 mg to 0.6 mg combined of DPPA, DPPC and PEG5000-DPPE per ml of solution.

The invention is based, in part, on the unexpected finding that lipid-encapsulated gas microspheres may be formed in sufficient quantities (to be useful as ultrasound contrast agents, for example) to provide a single patient dose using (a) a small volume of lipid solution and large gas headspace (relative to the total container volume), (b) a low lipid concentration, and/or (c) containers of different shape and size (volume), without affecting the average (or mean) diameter of the microspheres from that of the clinically useful ultrasound contrast agent, DEFINITY^{®}, and thus without compromising the acoustic properties of the microspheres. The ability to form lipid-encapsulated gas microspheres suitable for clinical use using substantially less lipid by reducing either the volume of lipid solution or lipid concentration is beneficial for a number of reasons, including reducing material wastage and the likelihood of overdosing a subject. The choice of container would allow the end user to select the most convenient shape and size (volume) for their desired application.

Thus, in one aspect, the invention provides a composition used to form an ultrasound contrast agent, comprising a lipid solution comprising DPPA, DPPC and PEG5000-DPPE, and a perfluorocarbon gas, in a container, wherein the perfluorocarbon gas occupies about 60-85% of the container volume, and wherein when activated the composition comprises microspheres having an average diameter of about 1 micron to about 2 microns (including about 1.0 micron to about 2.0 microns).

In another aspect, the invention provides a composition for use as an ultrasound contrast agent, comprising lipid-encapsulated gas microspheres having an average diameter ranging from about 1.0 micron to about 2.0 microns in a mixture of a lipid solution and a perfluorocarbon gas in a container, wherein the perfluorocarbon gas occupies about 60-85% of the container volume.

In another aspect, the invention provides a composition used to form an ultrasound contrast agent, comprising a lipid solution comprising DPPA, DPPC and PEG5000-DPPE, and a perfluorocarbon gas, in a container, wherein the perfluorocarbon gas occupies about 60-85% of the container volume.

In another aspect, the invention provides a composition for use as an ultrasound contrast agent, comprising lipid-encapsulated gas microspheres in a mixture of a lipid solution comprising DPPA, DPPC and PEG5000-DPPE and a perfluorocarbon gas in a container, wherein the perfluorocarbon gas occupies about 60-85% of the container volume.

In another aspect, the invention provides a composition used to form an ultrasound contrast agent, comprising a lipid solution comprising DPPA, DPPC and PEG5000-DPPE, and a perfluorocarbon gas, in a container, wherein the perfluorocarbon gas occupies about 60-85% of the container volume, and wherein when activated the composition comprises about 0.5 × 10⁹ to about 3.5 × 10⁹ microspheres per mL.

In another aspect, the invention provides a composition for use as an ultrasound contrast agent, comprising about 0.5 × 10⁹ to about 3.5 × 10⁹ lipid-encapsulated gas microspheres per mL in a mixture of a lipid solution comprising DPPA, DPPC and PEG5000-DPPE and a perfluorocarbon gas in a container, wherein the perfluorocarbon gas occupies about 60-85% of the container volume.

In another aspect, the invention provides a composition used to form an ultrasound contrast agent, comprising a lipid solution comprising about 0.1 mg to about 0.6 mg of DPPA, DPPC and PEG5000-DPPE (combined) per ml of solution, and a perfluorocarbon gas, in a container, wherein when activated the composition comprises microspheres having an average diameter of about 1 micron to about 2 microns (including about 1.0 micron to about 2.0 microns).

In another aspect, the invention provides a composition for use as an ultrasound contrast agent, comprising lipid-encapsulated gas microspheres having an average diameter ranging from about 1.0 micron to about 2.0 microns in a mixture of a lipid solution and a perfluorocarbon gas in a container, wherein the lipid solution comprises about 0.1 to about 0.6 mg lipid per ml of solution.

The invention provides a composition used to form an ultrasound contrast agent, comprising a lipid solution comprising about 0.1 mg to about 0.6 mg combined of DPPA, DPPC and PEG5000-DPPE per ml of solution, and a perfluorocarbon gas, in a container.

The invention provides a composition for use as an ultrasound contrast agent, comprising lipid-encapsulated gas microspheres in a mixture of a lipid solution and a perfluorocarbon gas in a container, wherein the lipid solution comprises about 0.1 to about 0.6 mg DPPA, DPPC and PEG5000-DPPE combined per ml of solution.

In another aspect, the invention provides a composition used to form an ultrasound contrast agent, comprising a lipid solution comprising about 0.1 mg to about 0.6 mg combined of DPPA, DPPC and PEG5000-DPPE per ml of solution, and a perfluorocarbon gas, in a container, wherein when activated the composition comprises about 0.1 × 10⁹ to about 3.5 × 10⁹ microspheres per mL.

In another aspect, the invention provides a composition for use as an ultrasound contrast agent, comprising about 0.1 × 10⁹ to about 3.5 × 10⁹ lipid-encapsulated gas microspheres per mL in a mixture of a lipid solution and a perfluorocarbon gas in a container, wherein the lipid solution comprises about 0.1 to about 0.6 mg DPPA, DPPC and PEG5000-DPPE combined per ml of solution.

In some embodiments, the microspheres (i.e., the detected or counted microspheres) have an average diameter ranging from about 1.2 microns to about 1.8 microns. In some embodiments, the microspheres (i.e., the detected or counted microspheres) have an average diameter of about 1.6 microns.

In some embodiments, at least 50% of the microspheres (i.e., detected or counted microspheres) have a diameter of about 1.0 to about 2.0 microns. That is, of the microspheres having a diameter in the range of 1-40 microns, at least 50% have a diameter of about 1.0 to about 2.0 microns. In some embodiments, of the microspheres having a diameter in the range of 1-40 microns (i.e., the detected microspheres), at least 70% have a diameter of about 1.0 to about 2.0 microns.

In some embodiments, the container is a vial. In some embodiments, the container is a tube. In some embodiments, the container is a syringe. Thus, in some embodiments, the container is a pre-loaded (or pre-filled) syringe. In some embodiments, the container is a v-bottom vial. In some embodiments, the container is a syringe lacking a rubber tip on the plunger. In some embodiments, the container is a plastic syringe having a rubber-less plunger. In some embodiments, the container is a plastic syringe having a substantially flat-end plunger.
In some embodiments, the container is a rubber-less container. In some embodiments, the container is a plastic container.

In some embodiments, the lipid solution comprises DPPA, DPPC and PEG5000-DPPE in a mole % ratio of 10:82:8. In some embodiments, the PEG5000-DPPE is MPEG5000-DPPE.

In some embodiments, the perfluorocarbon gas is perfluoropropane. In some embodiments, the perfluorocarbon gas occupies about 65%, about 70%, about 75%, about 80% or about 85% of the volume of the container.

In some embodiments, the lipid solution further comprises propylene glycol, glycerin (i.e., glycerol) and saline. In some embodiments, the lipid solution comprises a buffer such as but not limited to a phosphate buffer. Thus, in some embodiments, the lipid solution further comprises propylene glycol, glycerin (i.e., glycerol), phosphate buffer, and saline. In some embodiments, the lipid solution further comprises saline, glycerin (i.e., glycerol) and propylene glycol in a weight ratio of 8:1:1. It is to be understood that the terms glycerin and glycerol are used interchangeably herein.

In some embodiments, the composition comprises about 1.76 ml lipid solution and about 2.03 ml of perfluorocarbon gas. In some embodiments, the composition comprises about 1 ml lipid solution and about 2.75 ml of perfluorocarbon gas.

In some embodiments, the lipid solution comprises about 0.1 to about 0.5 mg of lipids per ml of solution. In some embodiments, the lipid solution comprises about 0.1 to about 0.4 mg of lipids per ml of solution. In some embodiments, the lipid solution comprises about 0.2 to about 0.5 mg of lipids per ml of solution. In some embodiments, the lipid solution comprises about 0.2 to about 0.4 mg of lipids per ml of solution. In some embodiments, the lipid solution comprises about 0.3 to about 0.4 mg of lipids per ml of solution. In some embodiments, the lipid solution comprises about 0.4 to about 0.5 mg of lipids per ml of solution. In some embodiments, the lipid solution comprises about 0.19 or about 0.2 mg lipids per ml of solution. In some embodiments, the lipid solution comprises about 0.38 or about 0.4 mg of lipids per ml of solution.

In another aspect, the invention provides a composition used to form an ultrasound contrast agent, comprising a lipid solution comprising DPPA, DPPC and PEG5000-DPPE, and a perfluorocarbon gas, in a container, wherein the perfluorocarbon gas occupies about 50-55% of the container volume, and wherein when activated the composition comprises microspheres having an average diameter of about 1 micron to about 2 microns (including about 1.0 micron to about 2.0 microns), wherein the container has a container volume of less than 3 mL and/or the container is a v-bottom container such as a v-bottom glass vial, or a rubber-less container such as a rubber-less syringe, optionally having a fixed or adjustable flat end. The concentrations of microspheres may range from about 0.1 to about 3.5 × 10⁹ microspheres/ml, including about 0.5 to about 3.5 × 10⁹ microspheres/ml. Various other embodiments recited above apply equally to this aspect of the invention. The microsphere number and concentration may be measured within minutes of activation of the compositions described herein, including within 60 minutes, within 30 minutes, or within 10 minutes, in some instances,

In another aspect, the invention provides a composition used to form an ultrasound contrast agent, comprising a lipid solution comprising DPPA, DPPC and PEG5000-DPPE, and a perfluorocarbon gas, in a container having an actual internal volume of less than 3 mL, wherein the perfluorocarbon gas occupies about 50-55% of the actual internal volume, and wherein when activated the composition comprises microspheres having an average diameter of about 1.0 micron to about 2.0 microns. In some embodiments, the actual internal volume is in the range of 1 mL to less than 3 mL.

In another aspect, the invention provides a composition for use as an ultrasound contrast agent, comprising lipid-encapsulated gas microspheres having an average diameter ranging from about 1.0 micron to about 2.0 microns in a mixture of a lipid solution comprising DPPA, DPPC and PEG5000-DPPE and a perfluorocarbon gas in a container having an actual internal volume of less than 3 mL, wherein the perfluorocarbon gas occupies about 50-55% of the actual internal volume. In some embodiments, the actual internal volume is 1 mL to less than 3 mL.

In another aspect, the invention provides a composition used to form an ultrasound contrast agent, comprising a lipid solution comprising DPPA, DPPC and PEG5000-DPPE, and a perfluorocarbon gas, in a rubber-less plastic container, optionally having an adjustable or fixed flat end, wherein the perfluorocarbon gas occupies about 50-55% of the container volume, and wherein when activated the composition comprises microspheres having an average diameter of about 1.0 micron to about 2.0 microns.

In another aspect, the invention provides a composition for use as an ultrasound contrast agent, comprising lipid-encapsulated gas microspheres having an average diameter ranging from about 1.0 micron to about 2.0 microns in a mixture of a lipid solution comprising DPPA, DPPC and PEG5000-DPPE and a perfluorocarbon gas in a rubber-less plastic container, optionally having an adjustable or fixed flat end, wherein the perfluorocarbon gas occupies about 50-55% of the container volume.

In another aspect, the invention provides a composition used to form an ultrasound contrast agent, comprising a lipid solution comprising DPPA, DPPC and PEG5000-DPPE, and a perfluorocarbon gas, in a glass container having a v-shaped bottom, wherein the perfluorocarbon gas occupies about 50-55% of the container volume, and wherein when activated the composition comprises microspheres having an average diameter of about 1.0 micron to about 2.0 microns.

In another aspect, the invention provides a composition for use as an ultrasound contrast agent, comprising lipid-encapsulated gas microspheres having an average diameter ranging from about 1.0 micron to about 2.0 microns in a mixture of a lipid solution comprising DPPA, DPPC and PEG5000-DPPE and a perfluorocarbon gas in a glass container having a v-shaped bottom, wherein the perfluorocarbon gas occupies about 50-55% of the container volume.

In other aspects, the invention provides methods for producing an ultrasound contrast agent by activating any of the foregoing compositions in order to form a population of lipid-encapsulated microspheres. The means for activation may vary depending on the container size (volume) and shape. In some embodiments, the composition may be activated for less than 5 minutes, less than 2 minutes, less than 1 minute, or less than 30 seconds, including for about 45 seconds or for about 20 seconds.

These and other aspects and embodiments of the invention will be described in greater detail herein.

### DETAILED DESCRIPTION OF THE INVENTION

The invention provides, inter alia, lipid-encapsulated gas microspheres and compositions thereof. The invention further provides methods of production of such microspheres and compositions to be used to form such microspheres.

As used herein, lipid-encapsulated gas microspheres are spheres having an internal volume that is predominantly gas and that is encapsulated by a lipid shell. The lipid shell may be arranged as a unilayer or a bilayer, including unilamellar or multilamellar bilayers. These microspheres are useful as ultrasound contrast agents.

The spheres have a diameter in the micron range. Preferably, the microspheres have an average diameter in the range of about 0.5 to about 2.5 microns, or about 1.0 to about 2.5 microns, more preferably in the range of about 1 to about 2 microns, even more preferably in the range of about 1.2 to about 1.8 microns, and most preferably in the range of about 1.4 to about 1.8 microns. An average diameter represents the average diameter of all detected microspheres in a composition. Microsphere diameter is typically measured using instrumentation such as that described herein (e.g., a Malvern FPIA-3000 Sysmex particle sizer). As will be understood in the art, such instrumentation typically has cutoff sizes for both the lower and upper limits. This means that microspheres below or above these cutoffs, respectively, are not counted (and are not included in the microsphere concentration calculation) and their diameter is not measured (and is not taken into consideration in determining the average diameter of microspheres). The instrumentation used in the Examples had a 1.0 micron lower limit cutoff and a 40.0 micron upper limit cutoff. In some embodiments, the microsphere average diameter is about 1.5 microns, or about 1.6 microns, or about 1.7 microns. In some embodiments, the microsphere average diameter is about 1.6 microns +/- 0.1 microns. These average diameters may also be expressed as the average diameter of detected microspheres (e.g., average diameter of microspheres having a diameter of at least 1.0 micron). The majority of counted or detected microspheres, using a lower cutoff of 1.0 micron and an upper cutoff of 40.0 microns, have a diameter in the range of 1.0 to 20.0 microns. It is also to be understood that this disclosure uses the terms microsphere size and microsphere diameter interchangeably. Thus, unless otherwise specified, microsphere size refers to microsphere diameter. Microsphere diameter may be measured for example using a Malvern FPIA-3000 Sysmex particle sizer, as shown in the Examples.

The invention further provides, inter alia, methods of making lipid-encapsulated gas microspheres of a particular size (diameter) range (e.g., the afore-mentioned ranges) using increased headspace gas volumes (relative to total container volume) which may also be represented as reduced lipid volume to gas volume ratios, compared to prior art methods. It was previously thought that increasing the headspace gas volume (or alternatively decreasing the lipid to gas volume ratio) would have a detrimental effect on microsphere formation, potentially resulting for example in microspheres having a diameter that differs from the clinically effective ultrasound contrast agent DEFINITY^{®}. However, it was unexpectedly found that the headspace gas volume could be increased (and thus the lipid to gas volume ratio could be decreased) without affecting microsphere diameter or markedly diminishing microsphere concentration, and thus importantly achieving a smaller preparation, without compromising in vivo utility as an ultrasound agent.

The invention further provides, inter alia, methods of making lipid-encapsulated gas microspheres of a particular size (diameter) range using less concentrated lipid solutions, compared to prior art methods. It was previously thought that decreasing lipid concentration would have a detrimental effect on microsphere formation, potentially resulting for example in microspheres having a diameter that differs from the clinically effective ultrasound contrast agent DEFINITY^{®}. However, it was unexpectedly found that the lipid concentration could be reduced without affecting microsphere diameter, and thus importantly without compromising in vivo utility as an ultrasound agent.

The compositions of the invention are considered improvements over existing ultrasound contrast agents. One such ultrasound contrast agent is marketed as DEFINITY^{®}. DEFINITY^{®} is an ultrasound contrast agent that is approved by the FDA for use in subjects with suboptimal echocardiograms to opacify the left ventricular chamber and to improve the delineation of the left ventricular endocardial border. DEFINITY^{®} is provided in a vial comprising a lipid solution comprising DPPA, DPPC and MPEG5000-DPPE in a 10:82:8 mole % ratio, and a headspace comprising perfluoropropane gas, in a Wheaton 2mL vial (Sulfate treated, Type I, borosilicate glass). Prior to its administration to a subject, DEFINITY^{®} is activated by vigorous shaking (and thereafter referred to as "activated DEFINITY^{®}"). Activation results in the formation of a sufficient number of lipid-encapsulated gas microspheres having an average diameter of 1.1 to 3.3 microns. DEFINITY^{®} is provided in a 2 ml glass vial having an actual internal volume of about 3.79 ml. The vial contains about 1.76 ml of lipid solution and a headspace gas volume of about 2.03 ml. Thus, the headspace volume (or gas volume) is about 54% of the total internal volume of the vial (when stoppered and sealed), representing a lipid to gas volume ratio of about 0.87. Each DEFINITY^{®} vial is provided as a single-use vial. However, the amount of lipid solution, and thus lipid-encapsulated gas microspheres, in the vial in some instances is more than is required for imaging a single subject, based on the package insert (FDA label) that instruct a user of the maximum patient dose. As a result, there is the possibility of overdosing a subject if all or nearly all the lipid contents of the vial are administered to the subject. In addition, there is the potential for material wastage.

The compositions of the invention overcome these problems by providing a sufficient number of microspheres for ultrasound imaging of most subjects using a significantly reduced amount of lipid. The invention accomplishes this by significantly altering the headspace gas volume (or the lipid to gas volume ratio). The effect of altering the headspace gas volume (or the lipid to gas volume ratio) as provided by the invention was previously reported to negatively impact microsphere size (diameter).

Unexpectedly, however, it was found in accordance with the invention that using a larger headspace gas volume (and thus a concomitant lower lipid to gas volume ratio) resulted in microspheres of similar size (diameter) and in similar concentrations to those obtained using marketed DEFINITY^{®}. As explained in greater detail herein and as exemplified in the Examples, increasing headspace gas volume from about 54% (as it exists in marketed DEFINITY^{®}) to about 73%, and concomitantly reducing the lipid to gas volume ratio from 0.87 to 0.36, surprisingly has no effect on microsphere diameter. Microspheres generated using a control "reconstituted" DEFINITY^{®} (containing about 1.76 ml lipid solution and 2.03 ml headspace gas, representing about a 54% headspace gas volume relative to the total container volume) had an average diameter of about 1.60 microns with a standard deviation of 0.04 microns, while microspheres generated using an altered composition (about 1.01 ml lipid solution and 2.78 ml headspace gas, representing about 73% headspace gas volume relative to the total container volume) had an average diameter of about 1.63 microns (standard deviation of 0.05 microns). Also surprising was the finding that other altered compositions having reduced lipid concentration compared to marketed DEFINITY^{®} also yielded lipid microspheres having an average diameter of 1.63 microns (standard deviation of 0.06 microns). Both findings are surprising because the art would have expected that such an increase in relative headspace gas volume or decrease in lipid concentration would result in marked effects on resultant lipid microspheres. See, for example, US Patent No. 5,656,211 which describes that increases in relative headspace gas volume result in larger microspheres and reductions in lipid concentration result in smaller microspheres. However, increasing relative headspace gas volume or reducing lipid concentration surprisingly had no significant effect on microsphere size (diameter).

Equally significantly and surprisingly, the microsphere concentration was similar between the two compositions with control "reconstituted" DEFINITY^{®} yielding a microsphere concentration of 2.36 × 10⁹ lipid microspheres/ml (standard deviation of 2.95 × 10⁸ lipid microspheres/ml) and the altered composition having an increased headspace gas volume yielding a microsphere concentration of 1.83 × 10⁹ lipid microspheres/ml (standard deviation of 3.93 × 10⁸ lipid microspheres/ml). The disclosure further provides that a sufficient number and thus concentration of microspheres of suitable diameter can be obtained even when starting with a lower lipid concentration. Thus, as demonstrated in the Examples, lipid-encapsulated gas microspheres generated according to the invention have equivalent acoustic properties to DEFINITY^{®}, thereby establishing that reductions in lipid content or lipid concentration during their production is not detrimental to their utility as ultrasound contrast agents.

The ability to generate compositions of lipid encapsulated gas microspheres that are still useful as ultrasound contrast agents using lower amounts of lipid is beneficial since it ensures the maximum amount of lipids (and other constituents) that could be administered to a subject from a single vial are reduced, thereby preventing overdoses.

The surprising finding that average microsphere diameters equivalent to DEFINITY^{®} could be achieved while reducing the lipid solution volume (and increasing headspace) or decreasing the lipid concentration was extended further to a smaller vial size. As explained in greater detail herein and as exemplified in the Examples, changing the vial volume from 3.79 mL (as exists for DEFINITY^{®}) to 2.9 mL (2 ml Schott) and keeping the lipid concentration (0.75 mg/mL) and headspace to container size ratio (about 54%) equivalent to DEFINITY^{®} produced microsphere size and concentration (microspheres per mL) equivalent to DEFINITY^{®}. In this smaller vial, increasing the headspace gas volume from 54% (as it exists in marketed DEFINITY^{®}) to 66%, and concomitantly reducing the lipid to gas volume ratio from 0.85 to 0.45, surprisingly has no effect on microsphere diameter.

Microspheres generated using a control "reconstituted" DEFINITY^{®} in a smaller vial (containing about 1.33 ml lipid solution and 1.57 ml headspace gas, representing a 54% headspace gas volume relative to the total container volume) had an average diameter of about 1.57 microns, while microspheres generated using an altered composition (about 0.9 ml lipid solution and 2.0 ml headspace gas, representing about a 69% headspace gas volume relative to the total container volume) had an average diameter of about 1.6 microns. Also surprising was the finding that other altered compositions having reduced lipid concentration (0.375 mg/mL), when compared to marketed DEFINITY^{®}, also yielded lipid microspheres having an average diameter of 1.66 and 1.67 microns with different headspace.

A further unexpected finding was the reduced microsphere concentration observed when a syringe with a rubber-tipped, cone shaped plunger was used as the container (e.g., as compared to a plastic tipped, substantially flat end shaped plunger). When the syringe with the rubber-tipped, cone-shaped plunger was used as the container (such as the Becton Dickinson 3 mL BD Luer Lok Tip, Cat. No. 309647), the resultant microsphere concentration (per mL) was reduced about 20-fold compared to the syringe with the plastic tipped, flat end plunger. The invention therefore contemplates the use of syringes having plungers that are not rubber-tipped and that optionally have substantially flat-ends in some embodiments. In some embodiments, the containers used to house the lipid solution, and within which the lipid solution is activated, do not comprise rubber material and may be referred to as rubber-less containers. Thus, in some instances, the containers may be rubber-less syringes or syringes comprising plungers that are not rubber-tipped. In some embodiments, the syringe is a latex-free syringe. In some embodiments, the syringe comprises no rubber and no silicon lubricants. In some embodiments, the syringe comprises no butyl rubber. In some embodiments, the container comprises no butyl rubber.

The compositions and methods of the invention will now be described in greater detail.

Certain compositions of the invention comprise a lipid solution and a gas in a container. The container therefore has a first volume and a second volume. The first volume is occupied by the lipid solution while the second volume is occupied by the gas. The gas volume may be referred to herein interchangeably as the headspace gas volume or the headspace volume, intending that the entire headspace volume is occupied by the gas of choice. It is to be understood, as described in greater detail herein, that the gas of choice may be a gas mixture such as a mixture of a perfluorocarbon gas and air.

The invention provides compositions in which the gas volume represents more than 60% of the total internal volume of the container. The relative gas volume may be about 60-95%, about 60-90%, about 60-85%, about 65-85%, about 70-85%, about 75-85%, or about 80-85%. The relative gas volume is the percentage of internal volume in the container that is occupied by gas. In some embodiments, the relative gas volume is about 70-75%, and in still other instances it is about 75+/-2%. In another instance, the relative gas volume is about 73%. As described in greater detail herein, the container may have a total internal volume of equal to or less than about 4 ml, including about 3.75 ml. The size of the container and its internal volume are not so limited, however.

In other aspects, the container may have an actual internal volume of less than 3 mL. In some embodiments, the container has an actual internal volume in the range of 1 mL to less than 3 mL. In some embodiments, the container has an actual internal volume of about 1.2 mL. It is to be understood that when ranges are recited herein, the value can be any value within the range and including the boundaries of the range. As an example, the volume range provided above means that the actual internal volume can be anywhere from 1 mL through to and including a volume that is less than 3 mL.

It will be understood in the art that a container such as a vial may be characterized by its actual internal volume. This is the maximum volume that could be housed in the container. Typically however the manufacturer of the container will suggest a smaller volume be placed into the vial and it is this volume that is sometimes used to describe the container (also referred to as the manufacturer's described usual fill). For example, the Wheaton 2 mL vial has an actual internal volume of about 3.9 mL but it is referred to as a 2 mL vial because it conveniently holds and thus tends to be used for about 2 mL liquid (rather than for example for 3.9 mL).

In some instances, compositions provided herein may also be described in terms of the ratio of the lipid volume (or lipid solution volume) and the gas volume. Thus, the invention provides for compositions having a first volume (i.e., lipid solution volume) to second volume (i.e., gas volume) ratio that is less than 0.87 (i.e., the quotient of lipid solution volume to headspace gas volume is less than 0.87). The first to second volume ratio may range from about 0.05 (intending 5% lipid to 95% gas volume) through to about 0.66 (intending 40% lipid to 60% gas volume).

The gas volume can be determined by knowing or measuring (1) the volume of lipid solution added to a container and (2) the total internal volume of the container. Thus, the gas volume (or the lipid to gas volume ratio) can be determined, even before the gas is added to the container and thus before agitating the composition in order to form the lipid encapsulated microspheres. Once the containers are closed and sealed (e.g., with stoppers and crimped collars), the lipid and gas volumes remain unchanged even after the composition has been agitated sufficiently to form lipid encapsulated gas microspheres. Prior to such agitation, the lipid solution is typically overlaid with the headspace gas, with an interface between the two phases in the container. Upon agitation however an emulsion is formed through the intermingling of the lipid and gas resulting in lipid encapsulation of the gas. Thus following agitation, when microspheres are present, the gas volume intends the total volume of gas in the microspheres and in the headspace.

Certain compositions are therefore prepared by placing a lipid solution in a container and then overlaying the gas. The gas may be overlaid by replacing an existing headspace gas, such as air, with a preferred gas such as a perfluorocarbon gas. An example of a perfluorocarbon gas is perfluoropropane. Gas exchangers suitable for this purpose are known in the art. An example of a gas exchange device is a lyophilizing chamber.

The container may be a vial such as a glass or plastic vial. The glass may be pharmaceutical grade glass. The container may be sealed with a stopper such as a rubber stopper. The container volume (i.e., the internal volume of the container) may be about 2-5 ml, preferably about 4 ml, even more preferably about 3.75 ml including 3.79 mL. An example of a suitable container is Wheaton 2 ml glass vial (Cat. No. 2802, B33BA, 2cc, 13 mm, Type I, flint tubing vial), having an actual internal volume of about 3.75 ml including 3.79 mL. An example of a suitable stopper is a West gray butyl lyo, siliconized stopper (Cat. No. V50, 4416/50, 13 mm). An example of a suitable seal is a West flip-off aluminum seal (Cat. No. 3766, white, 13 mm). The container may be a Wheaton 1mL V-vial (Cat. No.W986214NG, Wheaton, 1 mL v-vial, Type I borosilicate glass). The container may be a 2 mL Schott vial (Cat. No.68000314, Schott 2 mL 13mm S/L FNT w/BB PF WOS). The container may be a tube. An exemplary tube is provided in the Examples. The container may be a syringe. Exemplary syringes are provided in the Examples. The containers are preferably sterile and/or are sterilized after introduction of the lipid solution and/or gas as described in published PCT application WO99/36104.

The lipid-encapsulated gas microspheres are formed in sufficient quantity by shaking the container. Shaking, as used herein, is defined as a motion that agitates an aqueous solution such that a gas is introduced from the headspace into the lipid solution. Any type of motion that agitates the lipid solution and results in the introduction of gas may be used for the shaking. The shaking must be of sufficient force to allow the formation of foam after a period of time. Preferably, the shaking is of sufficient force such that foam is formed within a short period of time, such as 30 minutes, and preferably within 20 minutes, and more preferably, within 10 minutes. In some embodiments, activation can occur in less than 5 minutes, less than 2 minutes, less than a minute, less than 50 seconds, less than 40 seconds, less than 30 seconds, or less than 20 seconds, including in about 45 seconds, in about 40 seconds, in about 30 seconds, or in about 20 seconds. The shaking may be by microemulsifying, by microfluidizing, for example, swirling (such as by vortexing), side-to-side, or up and down motion. Different types of motion may be combined. The shaking may occur by shaking the container holding the lipid solution, or by shaking the lipid solution within the container without shaking the container itself. Further, the shaking may occur manually or by machine. Mechanical shakers that may be used include, for example, a shaker table, such as a VWR Scientific (Cerritos, Calif.) shaker table, a microfluidizer, Wig-L-Bug^{™} (Crescent Dental Manufacturing, Inc., Lyons, Ill.), and a mechanical paint mixer. Vigorous shaking is defined as at least about 60 shaking motions per minute. This is preferred in some instances. Vortexing at at least 1000 revolutions per minute is an example of vigorous shaking and is more preferred in some instances. Vortexing at 1800 revolutions per minute is even more preferred in some instances.

Another suitable shaking device is VIALMIX^{®} which is described in U.S. Patent No. 6,039,557. Containers such as vials may be sufficiently agitated using VIALMIX^{®} for the ranges of times recited above, including for example 45 seconds.

It will be understood that the manner of activation may depend on the type and size of container and the optimal activation (e.g., shake) time may differ for different sized and shaped containers.

After activation, the composition typically appears as a milky white solution from which a volume may be drawn, optionally diluted, and administered to a subject as either a bolus or a continuous injection.

The gas is preferably substantially insoluble in the lipid solution of the compositions. The gas may be a non-soluble fluorinated gas such as sulfur hexafluoride or a perfluorocarbon gas. Examples of perfluorocarbon gases include perfluoromethane, perfluoroethane, perfluorobutane, perfluoropentane, perfluorohexane. Examples of gases that may be used in the microspheres of the invention are described in US Patent No. 5656211 An important embodiments, the gas is perfluoropropane. The headspace of a container such as a vial may contain, in some instances, about 3 mg to about 8 mg/ml, or about 4 mg to about 7 mg/ml, or about 5 mg to about 7 mg/ml or about 6 mg to about 7 mg/ml perfluoropropane (also known as octafluoropropane). In some embodiments, the headspace of the container may contain about 6.52 mg/ml perfluoropropane.

As used herein, a lipid solution is an aqueous solution comprising a mixture of lipids. In important embodiments, the lipid(s) may be phospholipid(s). The lipids may be cationic, anionic or neutral lipids.

The lipids may be of either natural, synthetic or semi-synthetic origin, including for example, fatty acids, fluorinated lipids, neutral fats, phosphatides, oils, fluorinated oils, glycolipids, surface active agents (surfactants and fluorosurfactants), aliphatic alcohols, waxes, terpenes and steroids.

Suitable lipids include, for example, fatty acids, lysolipids, fluorinated lipids, phosphocholines, such as those associated with platelet activation factors (PAF) (Avanti Polar Lipids, Alabaster, Ala.), including 1-alkyl-2-acetoyl-sn-glycero 3-phosphocholines, and 1-alkyl-2-hydroxy-sn-glycero 3-phosphocholines; phosphatidylcholine with both saturated and unsaturated lipids, including dioleoylphosphatidylcholine; dimyristoyl-phosphatidylcholine; dipentadecanoylphosphatidylcholine; dilauroylphosphatdylcholine; 1,2-dipalmitoyl-sn-glycero-3-phosphatidylcholine (DPPC); distearoylphosphatidylcholine (DSPC); and diarachidonylphosphatidylcholine (DAPC); phosphatidylethanolamines, such as dioleoyl-phosphatidylethanolamine, 1,2-dipalmitoyl-sn-glycero-3-phosphatidylethanolamine (DPPE) and distearoyl-phosphatidylethanolamine (DSPE); phosphatidylserine; phosphatidylglycerols, including distearoylphosphatidylglycerol (DSPG); phosphatidylinositol; sphingolipids such as sphingomyelin; glycolipids such as ganglioside GM1 and GM2; glucolipids; sulfatides; glycosphingolipids; phosphatidic acids, such as 1,2-dipalmitoyl-sn-glycero-3-phosphatidic acid (DPPA) and distearoylphosphatidic acid (DSPA); palmitic acid; stearic acid; arachidonic acid; and oleic acid.

The most preferred lipids are phospholipids, preferably 1,2-dipalmitoyl-sn-glycero-3-phosphatidylcholine (DPPC); 1,2-dipalmitoyl-sn-glycero-3-phosphatidic acid (DPPA); and 1,2-dipalmitoyl-sn-glycero-3-phosphatidylethanolamine (DPPE). DPPA and DPPE may be provided as monosodium salt forms.

In some instances, the lipid components may be modified in order to decrease the reactivity of the microsphere with the surrounding environment, including the in vivo environment, thereby extending its half-life. Lipids bearing polymers, such as chitin, hyaluronic acid, polyvinylpyrrolidone or polyethylene glycol (PEG), may also be used for this purpose. Lipids conjugated to PEG are referred to herein as PEGylated lipids. Preferably, the PEGylated lipid is DPPE-PEG or DSPE-PEG.

Conjugation of the lipid to the polymer such as PEG may be accomplished by a variety of bonds or linkages such as but not limited to amide, carbamate, amine, ester, ether, thioether, thioamide, and disulfide (thioester) linkages.

Terminal groups on the PEG may be, but are not limited to, hydroxy-PEG (HO-PEG) (or a reactive derivative thereof), carboxy-PEG (COOH-PEG), methoxy-PEG (MPEG), or another lower alkyl group, e.g., as in iso-propoxyPEG or t-butoxyPEG, amino PEG (NH2PEG) or thiol (SH-PEG).

The molecular weight of PEG may vary from about 500 to about 10000, including from about 1000 to about 7500, and from about 1000 to about 5000. In some important embodiments, the molecular weight of PEG is about 5000. Accordingly, DPPE-PEG5000 or DSPE-PEG5000 refers to DPPE or DSPE having attached thereto a PEG polymer having a molecular weight of about 5000.

The percentage of PEGylated lipids relative to the total amount of lipids in the lipid solution, on a molar basis, is at or between about 2% to about 20%. In various embodiments, the percentage of PEGylated lipids relative to the total amount of lipids is at or between 5 mole percent to about 15 mole percent.

In some embodiments where DPPA, DPPC and DPPE are used, their molar percentages may be about 77-90 mole % DPPC, about 5-15 mole % DPPA, and about 5-15 mole % DPPE, including DPPE-PEG5000. In some embodiments, the mole % ratio of DPPC, DPPA and DPPE including DPPE-PEG5000 is 82:10:8 respectively.

The lipid concentration in the lipid solution may vary depending on the embodiment. In some embodiments, the lipid concentration may be about 0.2 mg to about 0.6 mg of lipid per ml of solution, or about 0.3 mg to about 0.5 mg of lipid per ml of solution. In some embodiments, the lipid concentration is about 0.35 mg to about 0.45 mg of lipid per ml of solution. In some embodiments, the lipid solution comprises about 0.19 or about 0.2 mg lipids per ml of solution. In some embodiments, the lipid solution comprises about 0.38 or about 0.4 mg of lipids per ml of solution. In comparative examples the lipid solution comprises about 0.75 mg of lipids per ml of solution.

Methods for making lipid solutions having these various combinations of lipids are described in detail in US Patent No. 5656211, in published PCT application WO99/36104, and in published US application US 2013/0022550.

The lipid solution may further comprise other constituents such as stabilizing materials or agents, viscosity modifiers, tonicity agents, coating agents, and suspending agents. Examples of each class of agents are known in the art and are provided in for example US Patent No. 5656211, in published PCT application WO99/36104, and in published US application US 2013/0022550.

In some important embodiments, the lipid solution comprises propylene glycol, glycerol (i.e., glycerin) and saline. Saline, glycerol (i.e., glycerin) and propylene glycol may be used in weight ratio ranges of 6-9.95 (saline) to 0.1 to 3 (glycerol) to 0.1 to 3 (propylene glycol). In some instances, the weight ratio may be a 8:1:1 weight ratio (saline:glycerol:propylene glycol, respectively).

The lipid solution may further comprise one or more buffers including but not limited to phosphate buffers. The pH of the solution may be about 6.2 to about 6.8.

In comparative examples, each ml of lipid solution comprises 0.75 mg of lipids (consisting of 0.045 mg DPPA, 0.401 mg DPPC, and 0.304 mg DPPE-PEG5000), 103.5 mg propylene glycol, 126.2 mg glycerol (i.e., glycerin), 2.34 mg sodium phosphate monobasic monohydrate, 2.16 mg sodium phosphate dibasic heptahydrate, and 4.87 mg sodium chloride in water.

In some embodiments, each ml of lipid solution comprises about 0.43 mg of lipids (consisting of 0.0225 mg DPPA, 0.2 mg DPPC, and 0.152 mg DPPE-PEG5000), 103.5 mg propylene glycol, 126.2 mg glycerol (i.e., glycerin), 2.34 mg sodium phosphate monobasic monohydrate, 2.16 mg sodium phosphate dibasic heptahydrate, and 4.87 mg sodium chloride in water.

The invention further provides methods of use of the microspheres and microsphere compositions. The microspheres are intended as ultrasound contrast agents, and they may be used in vivo in human or non-human subjects or in vitro. The compositions of the invention may be used for diagnostic or therapeutic purposes or for combined diagnostic and therapeutic purposes.

When used as ultrasound contrast agents for human subjects, the compositions are activated as described herein in order to form a sufficient number of microspheres, optionally diluted into a larger volume, and administered in one of more bolus injections or by a continuous infusion. Administration is typically intravenous injection. Imaging is then performed shortly thereafter. The imaging application can be directed to the heart or it may involve another region of the body that is susceptible to ultrasound imaging such as but not limited to tumors or other abnormal growths and masses. Subjects of the invention include but are not limited to humans and animals. Humans are preferred in some instances.

The lipid compositions are administered in effective amounts. An effective amount will be that amount that facilitates or brings about the intended in vivo response and/or application. In the context of an imaging application, such as an ultrasound application, the effective amount may be an amount of lipid microspheres that allow imaging of a subject or a region of a subject.

### EXAMPLES

### Example 1.

Vials of DEFINITY^{®} manufactured by Lantheus Medical Imaging, Inc. contained the following phospholipids: 1,2-dipalmitoyl-sn-glycero-3-phosphatidylcholine (DPPC; 0.401 mg/mL), 1,2-dipalmitoyl-sn-glycero-3-phosphatidic acid (DPPA; 0.045 mg/mL), and N-(methoxypolyethylene glycol 5000 carbamoyl)-1,2-dipalmitoyl-sn-glycero-3-phosphatidylethanolamine (MPEG5000 DPPE; 0.304 mg/mL) in a matrix of 103.5 mg/mL propylene glycol, 126.2 mg/mL glycerol (i.e., glycerin), and 2.34 mg/mL sodium phosphate monobasic monohydrate, 2.16 mg/mL sodium phosphate dibasic heptahydrate, and 4.87 mg/mL sodium chloride in Water for Injection. The pH is 6.2-6.8.

The volume of the lipid solution was approximately 1.76 mL in a 2cc Wheaton glass vial with an actual internal volume of 3.79 mL and a head space of approximately 2.03 containing perfluoropropane gas (PFP, 6.52 mg/mL).

Vials had either A) 0.75 mL of the lipid solution removed and replaced with PFP gas, B) 0.75 mL of the lipid solution removed and replaced with matrix only (i.e., no DPPA, no DPPC and no DPPE) or C) 0.75 mL of the lipid solution removed and replaced with lipid solution to reconstitute DEFINITY^{®} (referred to herein as "reconstituted" DEFINITY^{®}). Vials were activated using a VIALMIX^{®} and a full 45 second shaking cycle. Five minutes after activation the microspheres were resuspended by 10 seconds of inversion by hand. Samples were removed using a syringe and 18 g needle in combination with a vent needle. These activation and handling procedures are consistent with the DEFINITY^{®} package insert.

Samples (0.1 mL) were analyzed using a Malvern FPIA-3000 Sysmex particle sizer and microsphere number and size (diameter) distribution were determined. This instrument was setup to measure microspheres in the range of ≥1 micron but ≤ 40 microns.

Acoustic attenuation was measured for selected samples using a Philips Sonos 5500 clinical ultrasound imaging system. Samples of vial types A, B or C were diluted 1:7.7 (1.3 ml plus 8.7 ml saline) in a 10 ml syringe. 200 microliter samples from this syringe were pipetted into a beaker containing 200 ml of 0.9% saline at room temperature. A 2 cm stirring bar maintained solution uniformity and the s3 transducer of the ultrasound system was positioned at the top of the beaker, just into the solution and 8.9 cm above the upper margin of the stirring bar. 5 seconds of 120 Hz images were then acquired digitally and written to disk. The ultrasound system was used in IBS mode, TGC was fixed at the minimal value for all depths, and LGC was disabled. The mechanical index (MI) was 0.2 with power set 18 dB below maximum. The receive gain was fixed at 90 and the compression at 0. For each sample tested ultrasound data acquisition was acquired prior to (blank) and after sample injection. Measurements were taken at 20, 60 and 120 seconds after introduction of the sample into the beaker, except for the single measurement of the C vial preparation for which the measurement was taken as soon as the image appeared visually uniform.

Image analysis was performed using Philips QLab, which read files produced by the ultrasound system and calculated values in dB for IBS mode. Regions of interest were drawn on the stirring bar and the dB values averaged over the full 5 second (approximately 360 video frame) acquisition. Attenuation measurements were obtained by subtracting the sample ROI value from the blank ROI value (both in dB). This was divided by twice the distance between the ultrasound transducer and the upper margin of the stirring bar to yield attenuation in dB/cm. Final values were obtained by applying a linear regression of the samples taken with respect to time after introduction to the beaker. The values used were derived from the intercept of the regression line with the y-axis. Means and standard deviations of these values were calculated, as appropriate, for samples from each of the vial types (A, B, or C as described above).

Testing of multiple type C vials containing 1.76 mL of a 0.75 mg/mL phospholipid solution and a 2.03 mLhead space (53.6% vial) had a mean 2.36×10⁹ microspheres/mL with a mean diameter of 1.60 µm (see Table 1). Maintaining the same headspace but decreasing the lipid concentration (type B vials) resulted in no meaningful change in mean microsphere size (diameter) (1.63 µm) but approximately halved the microsphere number and concentration. Maintaining the lipid concentration but decreasing the lipid volume and increasing the head space to 73% of vial (type A vials) resulted in no change in microsphere mean size (diameter) (1.63 µm) and a microsphere concentration close to type C vials (2.36×10⁹ vs 1.83×10⁹).

**Table 1. Microsphere size (diameter) distribution**

| | **A: 1.01 mL of 0.75 mg/mL lipid and 2.78 mL headspace** | | | **B: 1.76 mL of 0.43 mg/mL lipid and 2.03 mL headspace** | | | **C: 1.76 mL of 0.75 mg/mL lipid and 2.03 mL headspace** | | |
|---|---|---|---|---|---|---|---|---|---|
| | Mean Dia. (µm) | Conc. (x 10⁹ microspheres per mL) | % of microsphere 1 to 2 µm | Mean Dia. (µm) | Cone. (x 10⁹ microspheres per mL) | % of microsphere 1 to 2 µm | Mean Dia. (µm) | Conc. (x 10⁹ microspheres per mL) | % of microsphere 1 to 2 µm |
| | 1.57 | 2.18 | 86.1 | 1.64 | 1.11 | 83.5 | 1.64 | 2.12 | 83.8 |
| | 1.63 | 1.79 | 82.7 | 1.74 | 0.86 | 79.9 | 1.61 | 2.04 | 84.7 |
| | 1.7 | 1.35 | 80.3 | 1.63 | 1.09 | 83.5 | 1.64 | 2.25 | 82.5 |
| | 1.62 | 1.84 | 83.5 | 1.60 | 1.44 | 86.4 | 1.57 | 2.54 | 86.8 |
| | 1.64 | 1.79 | 83.0 | 1.56 | 1.39 | 87.9 | 1.58 | 2.84 | 86.4 |
| | 1.7 | 1.39 | 79.9 | 1.59 | 1.10 | 85.8 | 1.56 | 2.39 | 87.4 |
| | 1.57 | 2.45 | 85.7 | | | | | | |
| | | | | | | | | | |
| **Mean** | 1.63 | 1.83 | 83.0 | 1.63 | 1.17 | 84.5 | 1.60 | 2.36 | 85.2 |
| **Std Dev** | 0.05 | 0.39 | 2.4 | 0.06 | 0.22 | 2.4 | 0.04 | 3.0 | 1.9 |
| **%RS D** | 3.27 | 21.47 | 2.9 | 3.84 | 18.50 | 3.3 | 2.20 | 12.49 | 2.2 |

Examination of microsphere samples from vial types A, B and C by ultrasound clinical imaging probe demonstrated acoustic attenuation/microsphere was very similar between different vial types (see Table 2). This indicates material from the three vial types will be equally effective for clinical imaging.

**Table 2. Acoustic attenuation (dB/cm/microsphere)**

| | **A: 1.01 mL of 0.75 mg/mL lipid and 2.78 mL headspace** | **B: 1.76 mL of 0.43 mg/mL lipid and 2.03 mL headspace** | **C: 1.76 mL of 0.75 mg/mL lipid and 2.03 mL headspace** |
|---|---|---|---|
| **Mean** | 9.91E-10 | 1.14E-09 | 8.20E-10 |
| **N** | 3 | 2 | 1 |

### Example 2. Activation of reduced volume

In other experiments, DEFINITY^{®} formulations with reduced lipid blend levels were tested. Vials were prepared with total phospholipid concentrations of 0.1875, 0.325, and 0.75 mg/mL, by diluting with a formulation matrix composed of phosphate buffer, in saline containing 10% v/v propylene glycol and 10% v/v glycerol (referred to as ¼, ½, and full concentration, respectively). Wheaton 2 cc vials were filled with 1.76 mL of the formulation being examined. The headspace of the vial was replaced with PFP, and the vial was sealed with a West grey butyl stopper and crimped with an aluminum seal. Vials were optimally activated and tested for microsphere number and average size (diameter) as described in Example 1. The data are provided in Table 3.

Reducing the phospholipid concentration resulted in a reduced number of microspheres per mL in a proportional manner. Microsphere diameter, however, was not significantly changed by the reduced concentration.

**Table 3. Microsphere concentration and diameter with reduced phospholipid concentration.^{a}**

| | **Microspheres per mL (× 10⁹)** | **Average Microsphere Diameter (µm)** | **% microspheres 1 to 2 µm** |
|---|---|---|---|
| **0.1875 mg/mL lipid (1/4 concentration)** | **0.54** | **1.75** | **78.1** |
| **0.375 mg/mL lipid (1/2 concentration)** | **1.38** | **1.66** | **82.2** |
| **0.75 mg/mL lipid (full concentration)** | **3.05** | **1.66** | **79.7** |

| | | | |
|---|---|---|---|
| ^{a}Vials were prepared with total phospholipid concentrations of 0.1875, 0.325, and 0.75 mg/mL, by diluting with a formulation matrix consisting of phosphate buffer in saline containing 10% v/v propylene glycol and 10% v/v glycerol at a total fill of 1.76 mL. The headspace air was replaced with PFP, the 2 cc Weaton vial sealed with a West grey butyl stopper, and vial crimped with an aluminum seal. Vials were optimally activated and tested for microsphere number and average size (diameter) as described in Example 1. | | | |

### Example 3. Effect of container shape and size (diameter)

DEFINITY^{®} lipid solution was filled into various containers including: vials, syringes, and pliable plastic tubes, and then activated. The containers examined all comprised a cylindrical shape with either a flat end or v-shaped end. The syringe and tubes were plastic and the syringes allowed adjustment of the cylindrical volumes by movement of the plunger. In all studies, an appropriate amount of phospholipid mixture (0.75 mg/mL) was placed in the container, the sample diluted if needed to achieve the desired phospholipid concentration, the headspace replaced with PFP, the container sealed, and the container/formulation activated. Microsphere numbers and mean diameter were determined as described above. Data are provided in Table 4, along with gas occupancy in each container.

**Table 4. Microsphere concentration for DEFINITY^{®} activated in various containers**

| | **Gas occupancy (%)** | **Microsphere Mean Diameter (microns)** | **Microsphere Concentration (× 10⁹ per mL)** | **% microspheres 1 to 2 µm** |
|---|---|---|---|---|
| **Wheaton V-vial^{a}** | 54 | 1.39 | 3.15 | 84.0 |
| **2 mL Schott, 1.33 mL 0.75 mg/mL lipid^{a}** | 54 | 1.57 | 3.00 | 85.5 |
| **2 mL Schott, 0.9 mL 0.75 mg/mL lipid^{b}** | 68 | 1.60 | 2.32 | 85.7 |
| **2 mL Schott,0.9 mL 0.375 mg/mL lipid^{b}** | 68 | 1.66 | 1.01 | 83.1 |
| **2 mL Schott, 1.33 mL of 0.375 mg/mL lipid^{b}** | 54 | 1.67 | 1.00 | 83.3 |
| **Tube 1.5 mL 0.75 mg/mL lipid^{c}** | Approximately 50 | 1.61 | 2.26 | 82.9 |
| **3 mL BD syringe, 0.75 mL of 0.75 mg/mL lipid^{d}** | Approximately 50 | 1.72 | 0.12 | 86.3 |
| **3 mL NORM-JECT syringe 0.75 mL of 0.75 mg/mL lipid^{e}** | Approximately 50 | 1.63 | 2.45 | 86.1 |
| **3 mL NORM-JECT syringe 0.45 mL of 0.75 mg/mL lipid^{e}** | Approximately 70 | 1.61 | 2.85 | 84.7 |
| **5 mL NORM-JECT syringe 2.25 mL of 0.75 mg/mL lipid^{f}** | Approximately 50 | 1.76 | 2.25 | 77.2 |

| | | | | |
|---|---|---|---|---|
| ^{a} DEFINITY^{®} lipid solution (0.75 mg/mL lipid) was filled into a 1 mL Wheaton V-vial, and a 2 mL Schott Glass Vial. The air headspace was replaced with PFP and the vial sealed with West grey butyl stoppers, crimped with an aluminum seal, activated and tested for microsphere number and average size as described in Example 1. The fill volumes of DEFINITY^{®} lipid solution were 0.55 and 1.33 mL for the Wheaton V-vial and 2 mL Schott Glass Vial, respectively. The total fillable volume for the Wheaton V-vial and 2 mL Schott Glass Vial was determined to be approximately 1.2 mL and 2.9 mL, respectively. Vials were activated at the optimal activation condition. using VIALMIX^{™}. ^{b} DEFINITY^{®} lipid solution (0.75 mg/mL lipid) (0.9 mL, 0.45 mL, and 0.665 mL) was filled into 2 mL Schott Glass Vials (total fillable volume 2.9 mL) and diluted with 0, 0.45 and 0.665 mL of formulation matrix without lipid blend, respectively. The air headspace was replaced with PFP and the vial sealed with West grey butyl stoppers, crimped with an aluminum seal, at the optimal activation condition using the VIALMIX^{™} and tested for microsphere number and average size as described in Example 1. ^{c} DEFINITY^{®}, lipid solution (0.75 mg/mL lipid) (1.5 mL) was filled into one chamber of a plastic, dual compartment, tube (NEOPAC Fleximed Tube, 13.5 × 80 mm, Hoffmann Neopac AG, Oberdiessbach, Switzerland). The air headspace was replaced with PFP and the tube folded creating approximately equal volumes of formulation and gas, taped to seal, activated and tested for microsphere number and average size as described in Example 1. Sample was activated using a WigLBug^{™} at the optimal activation condition. ^{d}DEPINITY^{®} lipid solution (0.75 mg/mL lipid) (0.75 mL) filled into a 3 mL Becton Dickinson (BD) syringe (Becton Dickinson Company (BD, Franklin Lake, NJ), 3 mL BD Luer Lok Tip Cat. No. 309647), and the air headspace was replaced with PFP to a gas volume of approximately 0.75 mL (PFP headspace was adjusted by positioning of the plunger to create the desired headspace gas occupancy), the syringe activated at the optimal activation condition using a VIALMIX^{™}, tested for microsphere number and average size as described in Example 1. ^{e}DEPINITY^{®} lipid solution (0.75 mg/mL lipid) (0.75 mL and 0.45 mL) filled into a 3 mL NORM-JECT^{®} syringe ((Henke-Sass, Wolf GmbH, Tuttlingen, Germany)), and the air headspace was replaced with PFP to a gas volume of approximately 0.75 mL and 1.05 mL, respectively (PFP headspace was adjusted by positioning of the plunger to create the desired headspace gas occupancy), the syringe activated with a VIALMIX^{™} at the optimal activation condition, tested for microsphere number and average size as described in Example 1. ^{f}DEPINITY^{®} lipid solution (0.75 mg/mL lipid) (2.25 mL) filled into a 5 mL NORM-JECT^{®} syringe (Henke-Sass, Wolf GmbH, Tuttlingen, Germany). The air headspace was replaced with PFP (PFP headspace was adjusted by positioning of the plunger to create a headspace ratio roughly equivalent to DEFINITY^{®}), the syringe activated with a WigLBug^{™} at the optimal activation condition, tested for microsphere number and average size as described in Example 1. | | | | |

This demonstrates that surprisingly a change in vial size (volume), shape and container composition still allowed, in most situations tested, appropriate mechanical agitation to produce microspheres with a microsphere diameter equivalent to DEFINITY^{®} and a microsphere concentration sufficient to allow ultrasound contrast imaging. This allows the container to be matched to the end user needs.

The one exception was the BD syringe, the use of which resulted in a 20-fold reduction in microsphere concentration. Surprisingly, the BD Syringe behaved differently from the NORM-JECT syringe. The BD syringe differs from the NORM-JECT syringe based on the plunger type and shape. The BD syringe comprises a rubber-tipped plunger having a relatively squat "cone" shaped end. In contrast, the NORM-JECT syringe comprises a plastic-tipped (i.e., not rubber-tipped) plunger (i.e., the end of the plunger is made from the same material as the remainder of the syringe barrel) having a substantially flat end.

## Claims

1. A composition used to form an ultrasound contrast agent, comprising
a lipid solution comprising DPPA, DPPC and PEG5000-DPPE, and
a perfluorocarbon gas, in a container,
wherein
the lipid solution comprises 0.1 mg to 0.6 mg combined of DPPA, DPPC and PEG5000-DPPE per ml of solution, and optionally wherein when activated
(i) the composition comprises microspheres having an average diameter of 1 micron to 2 microns, or/and
(ii) the composition comprises 0.1 × 10⁹ to 3.5 × 10⁹ microspheres per mL.

2. An ultrasound contrast agent composition comprising
lipid-encapsulated gas microspheres in a mixture of a lipid solution comprising DPPA, DPPC and PEG5000-DPPE and a perfluorocarbon gas in a container, wherein
the lipid solution comprises 0.1 to 0.6 mg DPPA, DPPC and PEG5000-DPPE (combined) per ml of solution and optionally wherein
(i) the lipid-encapsulated gas microspheres have an average diameter ranging from 1.0 micron to 2.0 microns, or/and
(ii) the composition comprises 0.1 × 10⁹ to 3.5 × 10⁹ lipid-encapsulated gas microspheres per mL.

3. The composition of claim 1, wherein the microspheres have an average diameter ranging from about 1.2 microns to about 1.8 microns, optionally wherein the microspheres have an average diameter of about 1.6 microns.

4. The composition of claim 1 or claim 3, wherein at least 50% of the microspheres have a diameter of about 1.0 to about 2.0 microns, optionally wherein at least 70% of the microspheres have a diameter of about 1.0 to about 2.0 microns.

5. The composition of any one of claims 1, 3 and 4, wherein the lipid solution comprises DPPA, DPPC and PEG5000-DPPE in a mole % ratio of 10:82:8.

6. The composition of any one of claims 1 and 3 to 5, wherein the PEG500-DPPE is MPEG5000-DPPE.

7. The composition of any one of claims 1 and 3 to 6, wherein the perfluorocarbon gas is perfluoropropane.

8. The composition of any one of claims 1 and 3 to 7, wherein the perfluorocarbon gas occupies about 65%, about 70%, about 75%, about 80% or about 85% of the volume of the container.

9. The composition of any one of claims 1 and 3 to 8, wherein the lipid solution further comprises propylene glycol, glycerol, and saline.

10. The composition of any one of claims 1 and 3 to 9, wherein the lipid solution further comprises propylene glycol, glycerol, buffer, and saline, optionally wherein the buffer is phosphate buffer.

11. The composition of any one of claims 1 and 3 to 10, wherein the lipid solution further comprises saline, glycerol and propylene glycol in a weight ratio of 8:1:1.

12. The composition of any one of claims 1 and 3 to 11, comprising about 1 ml lipid solution and about 2.75 ml of perfluorocarbon gas.

13. The composition of any one of claims 1, and 3 to 11, wherein the composition comprises about 1.76 ml lipid solution and about 2.03 ml of perfluorocarbon gas.

14. The composition of any one of claims 1, 3 to 11 and 13, wherein the lipid solution comprises about 0.2 to about 0.5 mg of lipids per ml of solution, preferably about 0.3 to about 0.4 mg of lipids per ml of solution, more preferably about 0.4 to about 0.5 mg of lipids per ml of solution.

15. The composition of any one of claims 1 and 3 to 14, wherein the container is a vial, a tube, or a syringe.

16. The composition of any one of claims 1 and 3 to 15, wherein the container is a rubber-less container.

17. The composition of any one of claims 1 and 3 to 16, wherein the container is a plastic container.

18. The composition of any one of claims 1 and 3 to 17, wherein the container is a plastic syringe having a rubber-less plunger.

19. The composition of any one of claims 1 and 3 to 18, wherein the container is a plastic syringe having a substantially flat-end plunger.

20. The composition of claim 1 comprising a lipid solution comprising DPPA, DPPC and PEG5000-DPPE, and a perfluorocarbon gas, in a container having an actual internal volume of less than 3 mL, wherein the perfluorocarbon gas occupies about 50-55% of the actual internal volume, and wherein when activated the composition comprises microspheres having an average diameter of about 1.0 micron to about 2.0 microns, wherein the actual internal volume is in the range of 1 mL to less than 3 mL.

21. A method for producing an ultrasound contrast agent, comprising
activating the composition of any one of claims 1 and 3 to 20 to form a population of lipid-encapsulated microspheres.

22. The method of claim 21, wherein the composition is activated for less than 1 minute, optionally wherein the composition is activated for about 20 to about 50 seconds.

23. The ultrasound contrast agent composition according to claim 2, wherein the composition comprises the additional features as defined in any one of claims 3 to 20.

## Patentansprüche

1. Zusammensetzung zur Verwendung zum Bilden eines Ultraschallkontrastmittels, umfassend
eine Lipidlösung, die DPPA, DPPC und PEG5000-DPPE umfasst, und
ein Perfluorkohlenstoffgas, in einem Behälter,
wobei
die Lipidlösung 0,1 mg bis 0,6 mg der Kombination von DPPA, DPPC und PEG5000-DPPE pro ml Lösung umfasst und wobei gegebenenfalls, wenn aktiviert,
(i) die Zusammensetzung Mikrokügelchen mit einem mittleren Durchmesser von 1 Mikrometer bis 2 Mikrometer umfasst und/oder
(ii) die Zusammensetzung 0,1 × 10⁹ bis 3,5 × 10⁹ Mikrokügelchen pro ml umfasst.

2. Ultraschallkontrastmittelzusammensetzung umfassend lipidverkapselte Gas-Mikrokügelchen in einem Gemisch einer Lipidlösung, die DPPA, DPPC und PEG5000-DPPE umfasst, und einem Perfluorkohlenstoffgas in einem Behälter, wobei
die Lipidlösung 0,1 bis 0,6 mg DPPA, DPPC und PEG5000-DPPE (kombiniert) pro ml Lösung umfasst, und wobei gegebenenfalls
(i) die lipidverkapselten Gas-Mikrokügelchen einen mittleren Durchmesser in dem Bereich von 1,0 Mikrometer bis 2,0 Mikrometer aufweisen oder/und
(ii) die Zusammensetzung 0,1 × 10⁹ bis 3,5 × 10⁹ lipidverkapselte Mikrokügelchen pro ml umfasst.

3. Zusammensetzung gemäß Anspruch 1, wobei die Mikrokügelchen einen mittleren Durchmesser in dem Bereich von etwa 1,2 Mikrometer bis etwa 1,8 Mikrometer aufweisen, gegebenenfalls wobei die Mikrokügelchen einen mittleren Durchmesser von etwa 1,6 Mikrometer aufweisen.

4. Zusammensetzung gemäß Anspruch 1 oder Anspruch 3, wobei wenigstens 50 % der Mikrokügelchen einen Durchmesser von etwa 1,0 bis etwa 2,0 Mikrometer aufweisen, gegebenenfalls wobei wenigstens 70 % der Mikrokügelchen einen Durchmesser von etwa 1,0 bis etwa 2,0 Mikrometer aufweisen.

5. Zusammensetzung gemäß einem der Ansprüche 1, 3 und 4, wobei die Lipidlösung DPPA, DPPC und PEG5000-DPPE in einem mol-%-Verhältnis von 10:82:8 umfasst.

6. Zusammensetzung gemäß einem der Ansprüche 1 und 3 bis 5, wobei das PEG500-DPPE MPEG5000-DPPE ist.

7. Zusammensetzung gemäß einem der Ansprüche 1 und 3 bis 6, wobei das Perfluorkohlenstoffgas Perfluorpropan ist.

8. Zusammensetzung gemäß einem der Ansprüche 1 und 3 bis 7, wobei das Perfluorkohlenstoffgas etwa 65 %, etwa 70 %, etwa 75 %, etwa 80 % oder etwa 85 % des Volumens des Behälters einnimmt.

9. Zusammensetzung gemäß einem der Ansprüche 1 und 3 bis 8, wobei die Lipidlösung ferner Propylenglycol, Glycerol und Kochsalzlösung umfasst.

10. Zusammensetzung gemäß einem der Ansprüche 1 und 3 bis 9, wobei die Lipidlösung ferner Propylenglycol, Glycerol, Puffer und Kochsalzlösung umfasst, wobei der Puffer gegebenenfalls Phosphatpuffer ist.

11. Zusammensetzung gemäß einem der Ansprüche 1 und 3 bis 10, wobei die Lipidlösung ferner Kochsalzlösung, Glycerol und Propylenglycol in einem Gewichtsverhältnis von 8:1:1 umfasst.

12. Zusammensetzung gemäß einem der Ansprüche 1 und 3 bis 11, umfassend etwa 1 ml Lipidlösung und etwa 2,75 ml Perfluorkohlenstoffgas.

13. Zusammensetzung gemäß einem der Ansprüche 1 und 3 bis 11, wobei die Zusammensetzung etwa 1,76 ml Lipidlösung und etwa 2,03 ml Perfluorkohlenstoffgas umfasst.

14. Zusammensetzung gemäß einem der Ansprüche 1, 3 bis 11 und 13, wobei die Lipidlösung etwa 0,2 bis etwa 0,5 mg Lipide pro ml Lösung, vorzugsweise etwa 0,3 bis etwa 0,4 mg Lipide pro ml Lösung, bevorzugter etwa 0,4 bis etwa 0,5 mg Lipide pro ml Lösung, umfasst.

15. Zusammensetzung gemäß einem der Ansprüche 1 und 3 bis 14, wobei der Behälter ein Fläschchen, ein Rohr oder eine Spritze ist.

16. Zusammensetzung gemäß einem der Ansprüche 1 und 3 bis 15, wobei der Behälter ein kautschukfreier Behälter ist.

17. Zusammensetzung gemäß einem der Ansprüche 1 und 3 bis 16, wobei der Behälter ein Kunststoffbehälter ist.

18. Zusammensetzung gemäß einem der Ansprüche 1 und 3 bis 17, wobei der Behälter eine Kunststoffspritze mit einem kautschukfreien Kolben ist.

19. Zusammensetzung gemäß einem der Ansprüche 1 und 3 bis 18, wobei der Behälter eine Kunststoffspritze mit einem Kolben mit einem im Wesentlichen flachen Ende ist.

20. Zusammensetzung gemäß Anspruch 1, umfassend eine Lipidlösung, die DPPA, DPPC und PEG5000-DPPE umfasst, und ein Perfluorkohlenstoffgas, in einem Behälter mit einem tatsächlichen Innenvolumen von weniger als 3 ml, wobei das Perfluorkohlenstoffgas etwa 50-55 % des tatsächlichen Innenvolumens einnimmt und wobei die Zusammensetzung, wenn aktiviert, Mikrokügelchen mit einem mittleren Durchmesser von etwa 1,0 Mikrometer bis etwa 2,0 Mikrometer umfasst, wobei das tatsächliche Innenvolumen in dem Bereich von 1 ml bis weniger als 3 ml liegt.

21. Verfahren zur Herstellung eines Ultraschallkontrastmittels, umfassend
Aktivieren der Zusammensetzung gemäß einem der Ansprüche 1 und 3 bis 20, um eine Population von lipidverkapselten Mikrokügelchen zu bilden.

22. Verfahren gemäß Anspruch 21, wobei die Zusammensetzung weniger als 1 Minute aktiviert wird, wobei die Zusammensetzung gegebenenfalls etwa 20 bis etwa 50 Sekunden aktiviert wird.

23. Ultraschallkontrastmittelzusammensetzung gemäß Anspruch 2, wobei die Zusammensetzung die zusätzlichen Merkmale gemäß einem der Ansprüche 3 bis 20 umfasst.

## Revendications

1. Composition utilisée pour former un agent de contraste ultrasonore, comprenant
une solution de lipides comprenant DPPA, DPPC et PEG5000-DPPE, et
un gaz de perfluorocarbure, dans un récipient,
la solution de lipides comprenant 0,1 mg à 0,6 mg combiné de DPPA, DPPC et PEG5000-DPPE par ml de solution, et éventuellement lorsqu'elle est activée
(i) la composition comprenant des microsphères ayant un diamètre moyen de 1 micron à 2 microns, ou/et
(ii) la composition comprenant 0,1 × 10⁹ à 3,5 × 10⁹ microsphères par mL.

2. Composition d'agent de contraste ultrasonore comprenant
des microsphères de gaz encapsulées par des lipides dans un mélange d'une solution de lipides comprenant DPPA, DPPC et PEG5000-DPPE et d'un gaz de perfluorocarbure dans un récipient,
la solution de lipides comprenant 0,1 à 0,6 mg de DPPA, DPPC et PEG5000-DPPE (combinés) par ml de solution, et éventuellement
(i) les microsphères de gaz encapsulées par des lipides ayant un diamètre moyen dans la plage de 1,0 micron à 2,0 microns, ou/et
(ii) la composition comprenant 0,1 × 10⁹ à 3,5 × 10⁹ microsphères de gaz encapsulées par des lipides par mL.

3. Composition selon la revendication 1, les microsphères ayant un diamètre moyen dans la plage d'environ 1,2 micron à environ 1,8 micron, éventuellement les microsphères ayant un diamètre moyen d'environ 1,6 micron.

4. Composition selon la revendication 1 ou la revendication 3, au moins 50 % des microsphères ayant un diamètre d'environ 1,0 à environ 2,0 microns, éventuellement au moins 70 % des microsphères ayant un diamètre d'environ 1,0 à environ 2,0 microns.

5. Composition selon l'une quelconque des revendications 1, 3 et 4, la solution de lipides comprenant DPPA, DPPC et PEG5000-DPPE en un rapport de % en moles de 10 : 82 : 8.

6. Composition selon l'une quelconque des revendications 1 et 3 à 5, le PEG500-DPPE étant MPEG5000-DPPE.

7. Composition selon l'une quelconque des revendications 1 et 3 à 6, le gaz de perfluorocarbure étant le perfluoropropane.

8. Composition selon l'une quelconque des revendications 1 et 3 à 7, le gaz de perfluorocarbure occupant environ 65 %, environ 70 %, environ 75 %, environ 80 % ou environ 85 % du volume du récipient.

9. Composition selon l'une quelconque des revendications 1 et 3 à 8, la solution de lipides comprenant en outre du propylèneglycol, du glycérol et une solution saline.

10. Composition selon l'une quelconque des revendications 1 et 3 à 9, la solution de lipides comprenant en outre du propylèneglycol, du glycérol, un tampon, et une solution saline, éventuellement le tampon étant un tampon phosphate.

11. Composition selon l'une quelconque des revendications 1 et 3 à 10, la solution de lipides comprenant en outre une solution saline, du glycérol et du propylèneglycol en un rapport en poids de 8 : 1 : 1.

12. Composition selon l'une quelconque des revendications 1 et 3 à 11, comprenant environ 1 ml de solution de lipides et environ 2,75 ml de gaz de perfluorocarbure.

13. Composition selon l'une quelconque des revendications 1 et 3 à 11, la composition comprenant environ 1,76 ml de solution de lipides et environ 2,03 ml de gaz de perfluorocarbure.

14. Composition selon l'une quelconque des revendications 1, 3 à 11 et 13, la solution de lipides comprenant environ 0,2 à environ 0,5 mg de lipides par ml de solution, préférablement environ 0,3 à environ 0,4 mg de lipides par ml de solution, plus préférablement environ 0,4 à environ 0,5 mg de lipides par ml de solution.

15. Composition selon l'une quelconque des revendications 1 et 3 à 14, le récipient étant un flacon, un tube ou une seringue.

16. Composition selon l'une quelconque des revendications 1 et 3 à 15, le récipient étant un récipient sans caoutchouc.

17. Composition selon l'une quelconque des revendications 1 et 3 à 16, le récipient étant un récipient de matière plastique.

18. Composition selon l'une quelconque des revendications 1 et 3 à 17, le récipient étant une seringue en matière plastique ayant un piston sans caoutchouc.

19. Composition selon l'une quelconque des revendications 1 et 3 à 18, le récipient étant une seringue en matière plastique ayant un piston à extrémité sensiblement plate.

20. Composition selon la revendication 1 comprenant une solution de lipides comprenant DPPA, DPPC et PEG5000-DPPE, et un gaz de perfluorocarbure, dans un récipient ayant un volume interne réel inférieur à 3 mL, le gaz de perfluorocarbure occupant environ 50 à 55 % du volume interne réel, et lorsqu'elle est activée la composition comprenant des microsphères ayant un diamètre moyen d'environ 1,0 micron à environ 2,0 microns, le volume interne réel étant dans la plage de 1 mL à moins de 3 mL.

21. Procédé pour la production d'un agent de contraste ultrasonore, comprenant
l'activation de la composition selon l'une quelconque des revendications 1 et 3 à 20 pour former une population de microsphères encapsulées par des lipides.

22. Procédé selon la revendication 21, la composition étant activée pendant moins de 1 minute, éventuellement la composition étant activée pendant environ 20 à environ 50 secondes.

23. Composition d'agent de contraste ultrasonore selon la revendication 2, la composition comprenant les caractéristiques supplémentaires telles que définies dans l'une quelconque des revendications 3 à 20.
